# EUROPEAN PATENT SPECIFICATION

(11) **EP 1 317 235 B1**
(45) Date of publication and mention of the grant of the patent: **01.03.2006**
(21) Application number: 01967883.8
(22) Date of filing: 13.09.2001
(51) Int. Cl.: A61F 13/64

(54) **ABSORBENT ARTICLE COMPRISING A WAIST BELT**
ABSORBIERENDER ARTIKEL MIT EINEM GÜRTEL
ARTICLE ABSORBANT COMPRENANT UNE CEINTURE A LA TAILLE

(30) Priority: 13.09.2000 SE 0003253
(43) Date of publication of application: 11.06.2003
(73) Proprietor: SCA Hygiene Products AB, 405 03 Göteborg (SE)
(72) Inventor: GUSTIN, Maria, S-416 72 Göteborg (SE); KARLSSON, Katharina, S-41195 Alingsas (SE)
(74) Representative: Egeröd, Lisbeth
(86) International application number: PCT/SE2001/001966
(87) International publication number: WO 2002/022065

(56) References cited:
- WO-A1-97/38658
- WO-A1-99/21522

## Description

### Technical field

The present invention refers to an absorbent article such as a diaper and an incontinence guard comprising a liquid permeable topsheet, a liquid impermeable backsheet and an absorbent body enclosed therebetween, said article having a front portion, a rear portion and a crotch portion therebetween, and further is provided with a pair of belt portions attached to the rear portion of the article and which are intended to be fastened together around the waist of the wearer and where said front portion is provided with attachment means intended to be attached to the belt portions, in such a way that the article will assume a pantlike shape, where the belt portions form a part of the waist portions of the pant.

### Background of the invention

Diapers and incontinence guards for incontinent adults usually have a garment portion holding an absorbent body in place against the user's body and attachment means which hold the garment portion in place also when the user is moving. A common type of attachment means are adhesive tapes or hook and loop fasteners of the touch-and-close type which directly attach the front and rear portions of the absorbent article to each other. It is further known, through e.g., EP-A-0 287 388, EP-A-0 409 307, EP-A-0 528 282, EP-A-0 605 012 and FR-A-2 586 558, to attach the front and rear portions of the article by means of a belt, at which the possibilities to adjust the fit are improved. The belt further provides a simplified change of diaper or incontinence guard, especially when the patient is standing up.

On a common type of belt diaper the belt portions are first attached around the waist of the patient and then the front portion of the diaper is attached to the outside of the belt using hook and loop fasteners or tape tabs, being arranged on the belt and /or the front portion. One problem is that the belt folds it self longitudinally upon usage, which leads to discomfort for the wearer.

WO 99/21522 describes a belt whose cross-section varies in stiffness. This leads to an increased comfort for the wearer. The belt adapt itself to some extent after the wearer since it is more flexible at the edges. However, the elements are longitudinally arranged in the belt which might lead to sagging of the belt anyhow.

It would therefore be desirable to provide a diaper or incontinence guard having a comfortable belt which fits persons having different sizes.

### Summary of the invention

The object of the present invention is to accomplish a belt to a diaper or incontinence guard being comfortable to wear and which will fit differently sized persons. This object has been solved in that the belt portions are provided with stiffening elements, being discontinuously arranged in the longitudinal direction of the belt, and whose largest extension is arranged essentially across the belt in relation to the longitudinal direction of the belt. These element makes the belt fully outstretched without sagging and distributes the force equally over the belt, which gives an increased comfort.

### Short description of drawings

The invention will in the following be closer described with reference to an embodiment shown in the accompanying drawings.

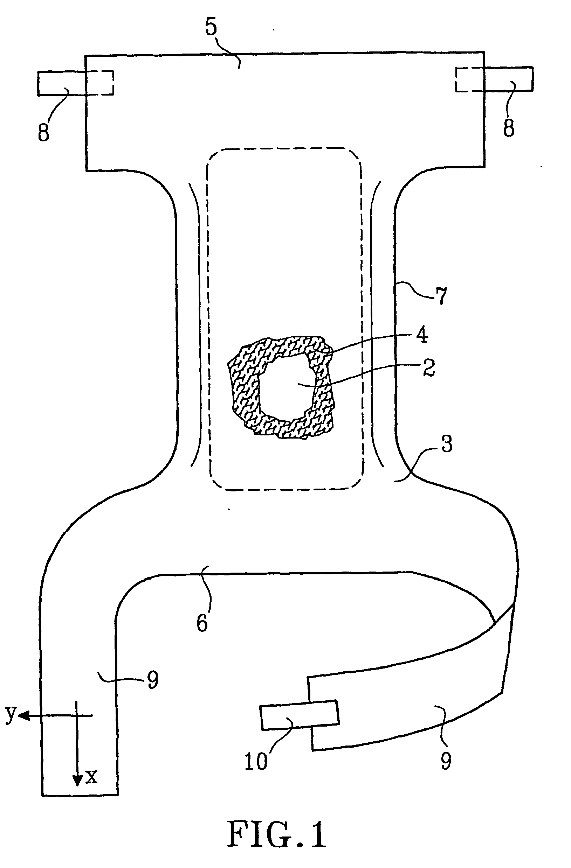

Fig. 1 shows schematically a perspective view of a diaper or incontinence guard according to the invention.
Fig. 2 shows examples of belts to the diaper in Fig. 1.

### Description of an embodiment

The drawing shows an embodiment of a diaper or incontinence guard 1 comprising a liquid impermeable backsheet 2, a liquid permeable topsheet 3 and an absorbent body 4 enclosed therebetween. The liquid permeable topsheet 3 can consist of a nonwoven material, e.g., a spunbond material of continuous filaments, a meltblown material, a bonded carded fibrous web or a perforated plastic film. The liquid impermeable backsheet 2 may consist of a plastic film, a nonwoven material coated with a liquid impervious material or a hydrophobic nonwoven material which resists liquid penetration.
The topsheet 3 and the backsheet material 2 have a somewhat greater extension in the plane than the absorbent body 4 and extend outside the edges thereof. The layers 2 and 3 are connected to each other within the projecting portions thereof, e.g., by gluing or welding by heat or ultrasonic.

The absorbent body 4 can be of any conventional kind. Examples of commonly occurring absorbent materials are cellulosic fluff pulp, tissue layers, highly absorbent polymers (so called superabsorbents), absorbent foam materials, absorbent nonwovens or the like. It is common to combine cellulosic fluff pulp with superabsorbents in an absorbent body. It is also common to have absorbent bodies comprising layers of different material with different properties with respect to liquid acquisition capacity, liquid distribution capacity and storage capacity. It is well-known to the person skilled in the art and does therefore not have to be described in detail. The thin absorbent bodies which are common in for example baby diapers and incontinence guards often comprise a compressed mixed or layered structure of cellulosic fluff pulp and superabsorbent.

The diaper/incontinence guard is intended to enclose the lower part of the wearer's trunk like a pair of absorbent pants. It comprises a front portion 5 intended during use to be worn on the front part of the user's body, a rear portion 6 intended during use to be worn on the rear part of the user's body, and a more narrow crotch portion 7 located between the front and rear portions and which is intended to be worn in the crotch part of the user between the legs. The front portion 5 is provides with a pair of adhesive tape portions 8 or other type of fastening means such as hooks and loop type fasteners.

A pair of belt portions 9 are with one end attached, e.g., glued or ultrasonically welded, to the rear portion 6 of the diaper. The belt portions 9 are with their opposite ends intended to be fastened together, e.g. by means of tape tab 10 which is taped against the outside of the opposite belt portion. Instead of tape tabs there may be another optional fastening means such as hook-and-loop type fasteners. The tape tabs 8 of the front portion 5 or corresponding fastening means are intended to be attached against the outside portions of the belt portions 9 in order to fasten together the diaper/incontinence guard to the desired pantlike shape.

According to an alternative embodiment the belt portions are attached to the front portion 5 of the diaper and thus are intended to be fastened together on the back of the wearer. The fastening means 8 are then arranged on the rear portion 6 of the diaper.

The width of the belt portions 9 should be between 5-20 cm, preferably between 7-15 cm.

The belt portions 9 are preferably a laminate of a carrier material, which forms the outside of the belt, and a soft nonwoven, which forms the inside of the belt intended to be in direct contact with the skin of the user. A suitable nonwoven material can be a spunbond material of e.g., polypropylene- or polyethylene fibres. Conjugate fibres may also be used. Another suitable nonwoven material can be a carded thermobonded material of e.g., polypropylene-, polyester- or conjugate fibres. As carrier material a plastic film or another suitable material e.g., nonwoven can be used. The carrier material should be adapted to function as a reception surface for the fastening means 8 and 10, wherein in the case where these fastening means comprise tape tabs a plastic film is suitable. In the case where other types of fastening means is used instead of tape tabs, e.g., hook and loop fasteners, another type of carrier material is used, which also may function as reception surface for the present fastening means. Also elastic laminates are suitable to use as material in the belt portions 9.

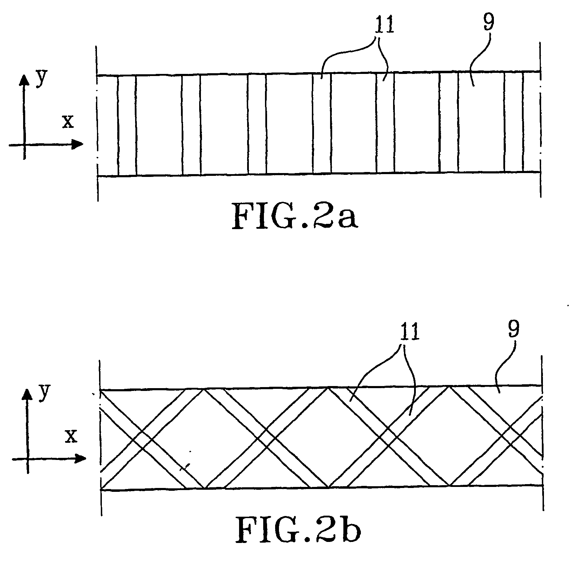

The belt portions 9 are reinforced with stiffening elements 11. These may be arranged across the belt having suitably adapted distances between said stiffening elements 11. The wording "across" used here, means that they extend between the opposite longitudinal edges of the belt portions 9, either essentially perpendicular to these edges or obliquely in relation to these. However, they do not have to extend all the way to the longitudinal edges of the belt portions 9, but may stop slightly inside these edges. It is however preferred that they extend to said longitudinal edges. The stiffening elements 11 may also form a pattern such as a striped pattern (Fig. 2a) or crosswise arranged pattern (Fig. 2b) or other suitable pattern being repeated in the longitudinal direction x of the belt. In the case where the stiffening elements are arranged in a striped pattern, the stripes may among each other have different widths in x direction, (x direction means here longitudinally to the belt). The stiffening elements 11 preferably include the same material already being in the belt portions 9 or in the rest of the diaper. Since the product is a disposable article, the material used should be intended for this purpose and not be to costly. The stiffening elements 11 may for instance consist of different kinds ofnonwoven material or plastic material and be obtained by adhesive joining, welding or melting of materials. A kind of a so-called hotmelt adhesive may also be used to obtain the stiffening elements 11. The stiffening elements 11 keep the belt extended in y direction (the expression y direction means here the width of the belt) in order to avoid that the belt sags, when it is being applied around the waist of the wearer. This makes the belt more comfortable to wear for the user.

The invention is of course not limited to the above described embodiment but can be modified within the scope of the claims which are hereto appended.

## Claims

1. Absorbent article such as a diaper and an incontinence guard comprising a liquid permeable topsheet (3), a liquid impermeable backsheet (2) and an absorbent body (4) enclosed therebetween, said article having a front portion (5), a rear portion (6) and a crotch portion (7) therebetween, and further is provided with a pair of belt portions (9) attached to the rear portion (6) alternatively the front portion (5) of the article and which are intended to be fastened together around the waist of the wearer and where said front portion (5) alternatively the rear portion (6) is provided with fastening means (8) intended to be attached to the belt portions (9), in such a way that the article will assume a pantlike shape when in use, where the belt portions (9) form a part of the waist portions of the pant and have a longitudinal direction (x) and a width direction (y),
**characterized in,**
**that** the belt portions (9) are provided with stiffening elements (11), being discontinuously arranged in the longitudinal direction (x) of the belt, and whose largest extension being arranged across the longitudinal direction (x) of the belt.

2. Absorbent article according to claim 1,
**characterized in,**
**that** the stiffening elements (11) being arranged in a pattern, being repeated along the longitudinal direction (x) of the belt.

3. Absorbent article according to claim 2,
**characterized in,**
**that** the stiffening elements (11) being arranged in a striped pattern.

4. Absorbent article according to claim 2,
**characterized in,**
**that** the stiffening elements (11) being arranged in a crosswise pattern.

## Patentansprüche

1. Absorbierender Gegenstand, wie beispielsweise eine Windel und ein Inkontinenzschutz, umfassend eine flüssigkeitsdurchlässige Oberlage (3), eine flüssigkeitsundurchlässige Decklage (2) und einen dazwischen eingeschlossenen Absorptionskörper (4), wobei der Gegenstand einen Vorderabschnitt (5), einen Hinterabschnitt (6) und einen Schrittabschnitt (7) dazwischen aufweist und ferner mit einem Paar Gürtelabschnitte (9) versehen ist, die an dem Hinterabschnitt (6) bzw. alternativ dem Vorderabschnitt (5) des Gegenstands angebracht sind und die dazu gedacht sind, um die Taille des Trägers befestigt zu werden und wobei der Vorderabschnitt (5) bzw. alternativ der Hinterabschnitt (6) mit einer Verschlusseinrichtung (8) versehen ist, die dazu gedacht ist, derart an den Gürtelabschnitten (9) angebracht zu werden, dass der Gegenstand im Gebrauch eine höschenähnliche Form annehmen wird, wobei die Gürtelabschnitte (9) einen Teil der Taillenabschnitte des Höschens bilden und eine Längsrichtung (x) und eine Breitenrichtung (y) aufweisen,
**dadurch gekennzeichnet,**
**dass** die Gürtelabschnitte (9) mit Versteifungselementen (11) versehen sind, die in der Längsrichtung (x) des Gürtels diskontinuierlich angeordnet sind und deren größte Dimension quer zur Längsrichtung (x) des Gürtels verläuft.

2. Absorbierender Gegenstand nach Anspruch 1,
**dadurch gekennzeichnet,**
**dass** die Versteifungselemente (11) in einem Muster angeordnet sind, das sich in der Längsrichtung (x) des Gürtels wiederholt.

3. Absorbierender Gegenstand nach Anspruch 2,
**dadurch gekennzeichnet,**
**dass** die Versteifungselemente (11) in einem gestreiften Muster angeordnet sind.

4. Absorbierender Gegenstand nach Anspruch 2,
**dadurch gekennzeichnet,**
**dass** die Versteifungselemente (11) in einem kreuzförmigen Muster angeordnet sind.

## Revendications

1. Article absorbant tel qu'une couche-culotte et une protection contre l'incontinence comprenant une feuille supérieure (3) perméable aux liquides, une feuille arrière (2) imperméable aux liquides et un corps absorbant (4) enfermé entre celles-ci, ledit article comportant une partie avant (5), une partie arrière (6) et une partie d'entrejambe (7) située entre celles-ci, et étant en outre muni d'une paire de parties ceinture (9) attachées à la partie arrière (6), ou en variante à la partie avant (5) de l'article, et qui sont destinées à être attachées ensemble autour de la taille de l'utilisateur, et ladite partie avant (5) ou en variante la partie arrière (6) étant munie de moyens d'attache (8) destinés à être fixés aux parties ceinture (9) de telle manière que l'article prendra, lors de son utilisation, une forme analogue à celle d'une culotte, les parties ceinture (9) constituant une portion des parties taille de la culotte et ayant une direction longitudinale (x) et une direction de largeur (y),
**caractérisé en ce que**
les parties ceinture (9) sont munies d'éléments de raidissement (11) qui sont agencés de façon discontinue dans la direction longitudinale (x) de la ceinture, et dont la plus grande étendue est agencée en travers de la direction longitudinale (x) de la ceinture.

2. Article absorbant selon la revendication 1,
**caractérisé en ce que**
les éléments de raidissement (11) sont agencés selon un motif qui est répété le long de la direction longitudinale (x) de la ceinture.

3. Article absorbant selon la revendication 2,
**caractérisé en ce que**
les éléments raidisseurs (11) sont agencés selon un motif en forme de bandes.

4. Article absorbant selon la revendication 2,
**caractérisé en ce que**
les éléments raidisseurs (11) sont disposés selon un motif en forme de croix.
